# EUROPÄISCHE PATENTSCHRIFT

(11) **EP 0 946 867 B1**
(45) Veröffentlichungstag und Bekanntmachung des Hinweises auf die Patenterteilung: **28.08.2002**
(21) Anmeldenummer: 97951858.6
(22) Anmeldetag: 18.12.1997
(51) Int. Cl.: G01N 27/30

(54) **ELEKTROCHEMISCHER SENSOR**
ELECTROCHEMICAL SENSOR
DETECTEUR ELECTROCHIMIQUE

(30) Priorität: 20.12.1996 DE 19653436
(43) Veröffentlichungstag der Anmeldung: 06.10.1999
(73) Patentinhaber: INSTITUT FUR CHEMO- UND BIOSENSORIK Münster E.V., 48149 Münster (DE)
(72) Erfinder: ADAM, Stefan, D-48565 Steinfurt (DE); BORCHARDT, Michael, D-48485 Neuenkirchen (DE); DIEKMANN, Christoph, D-48149 Münster (DE); STEINKUHL, Ralf, D-48155 Münster (DE)
(74) Vertreter: Pfenning, Meinig & Partner
(86) Internationale Anmeldenummer: DE9702960
(87) Internationale Veröffentlichungsnummer: WO98028614

(56) Entgegenhaltungen:
- EP-A- 0 266 432
- EP-A- 0 592 805
- US-A- 5 538 620

## Beschreibung

Die Erfindung betrifft einen Sensor zur Bestimmung von Stoffkonzentrationen, Aktivitäten oder zum Stoffnachweis auf der Basis von elektrochemischen Reaktionen.

Ein bei elektrochemischen Sensoren häufig verwendeter Aufbau besteht aus einem planaren Träger, auf welchem eine planare Nachweiselektrode angeordnet ist. Diese Elektrode befindet sich in Kontakt mit dem Probenmedium. Infolge elektrochemischer Reaktionen an der Elektrodenoberfläche können bestimmte Stoffe, bei Platineletroden beispielsweise Wasserstoffperoxid, elektrochemisch nachgewiesen werden.

Nachteilig bei derartigen Sensoranordnungen ist die vergleichsweise geringe Elektrodenoberfläche und die daraus resultierenden oftmals geringen Stromdichten. Insbesondere im Zuge der fortschreitenden Miniaturisierung stellen die zur Erzielung ausreichend hoher Stromdichten benötigten Oberflächenabmessungen planarer Elektroden oftmals eine unüberwindbare Grenze zu kleineren Sensorbauformen hin dar.

Häufig steht die planare Sensorelektrode in Kontakt mit einer stofferkennenden Substanz in Form einer dünnen Membran. In der Membran werden infolge einer spezifischen Nachweisreaktion Stoffe gebildet, welche an der Elektrode elektrochemisch nachgewiesen werden können.

Eine dünne Membran gewährleistet kurze Diffusionswege vom Ort der chemischen Nachweisreaktion zur Elektrode und damit kurze Ansprechzeiten. Ein weiterer Vorteil dünner Membranen ist außerdem die Vermeidung von Substratlimitierungen. Bei Glukosesensoren auf der Basis von Glukoseoxidase beispielsweise besteht die Gefahr einer Sauerstofflimitierung und damit von unerwünschten Nichtlinearitäten, wenn der für die Nachweisreaktion erforderliche Sauerstoff nicht in ausreichender Menge in die Membran eindiffundieren kann.

Diese Vorzüge dünner stofferkennender Membranen werden allerdings teilweise durch Haftungsprobleme aufgrund der oftmals schwierigen Anbindung der dünnen Membran an Träger und Elektrode sowie durch Verkapselungsprobleme kompensiert. Ein weiterer Nachteil von dünnen Membranen ist die kurze Lebensdauer derartiger Sensoren, da die aufgrund des geringen Membranvolumens vergleichsweise wenigen aktiven Membrankomponenten in kurzer Zeit desaktiviert oder verbraucht werden.

Die zuletzt genannten Nachteile lassen sich zum Teil durch die Verwendung dickerer stofferkennender Membranen vermeiden. Bei dickeren Membranen ergibt sich jedoch das Problem, daß aufgrund der langen Diffusionswege vom Ort der chemischen Umsetzung zur auf dem Träger angeordneten planaren Elektrode lange Ansprechzeiten des Sensors in Kauf genommen werden müssen. Desweiteren kann ein Teil der umgesetzten Stoffe vor der Detektion an der Elektrodenoberfläche aus der stofferkennenden Membran diffundieren, wodurch die Sensitivität herabgesetzt wird.

In der EP 0 603 154 A2 wird eine amperometrische Enzymelektrode zur Messung der Konzentration eines Enzymsubstrates in einer Probe mit einem auf bzw. in einem elektrisch leitenden, porösen Elektrodenmaterial immobilisierten bzw. adsorbierten Enzym beschrieben, wobei das Elektrodenmaterial einen redoxinaktiven Leiter mit einem leitfähigen Pigment und einem selbst nichtleitenden Bindemittel und eine darin fein verteilte katalytische Substanz aufweist.

In der EP 0 247 850 A1 erfolgt eine Unterdrückung von Interferenzen, die auf redoxaktive Metabolite und Medikamente wie z.B. p-Azetamol, Ascorbat oder Harnsäure zurückzuführen sind. Durch den Einsatz von Elektroden, bei denen fein verteilte Partikel der Platinmetalle auf der Oberfläche einer porösen Schicht bestehend aus Graphit und einem porösen polymeren Bindemittel aufgebracht sind.

Unabhängig von der Membrandicke tritt häufig ein Problem mit Sensorquerempfindlichkeiten auf. Die erwünschte Minimierung derartiger Querempfindlichkeiten geht häufig mit aufwendigen und daher teuren Modifikationen gebräuchlicher Sensoren einher.

Der Erfindung liegt die Aufgabe zugrunde, die Nachteile des Standes der Technik zu überwinden und einen miniaturisierbaren elektrochemischen Sensor zur Analyse flüssiger oder gasförmiger Proben zu schaffen, welcher insbesondere in Kombination mit stofferkennenden Substanzen kurze Ansprechzeiten und gleichzeitig einen vergrößerten linearen Meßbereich sowie eine lange Lebensdauer aufweist.

Diese Aufgabe wird gelöst durch die kennzeichnenden Merkmale des Anspruches 1. Vorteilhafte Ausgestaltungen und Weiterbildungen der Lösung ergeben sich aus den Unteransprüchen.

Bei einem elektrochemischen Sensor mit einem Träger und mindestens einer im Bereich des Trägers angeordnete Elektrode mit inneren Hohlräumen, in welche der zu bestimmende Stoff und/oder umgesetzte Reaktionsprodukte eintreten können, und bei dem in diese Hohlräume zumindest bereichsweise eine stofferkennende Substanz eingelagert ist, lassen sich auch bei geringen äußeren Elektrodenabmessungen vorteilhaft große aktive Nachweisoberflächen im Elektrodeninneren erzielen. Ein derartiger Sensor gestattet die Realisierung hoher Stromdichten und eignet sich inbesondere auch für die Miniaturisierung.

Elektroden mit inneren Hohlräumen und demzufolge mit inneren Oberflächen weisen im Gegensatz zu den herkömmlichen planaren Elektroden eine im wesentlichen dreidimensionale Funktionalität auf. Derartige Elektroden können beispielsweise einen gitter-, netz-, fadenförmigen oder porösen Aufbau aufweisen. Wichtig ist, daß im Innern der Elektrode Poren, Schläuchen oder andere Hohlräume mit Oberflächen vorhanden sind, über welche das zu analysierende Medium und der nachzuweisende Stoff oder umgesetzte Reaktionsprodukte des nachzuweisenden Stoffes mit der Elektrodenoberfläche in Kontakt treten können.

Bei einem Sensor mit in die Sensorelektrode eingelagerter stofferkennender Substanz lassen sich die Vorteile von Sensoren auf der Basis von Dünnschichtmembranen und von solchen auf der Basis von Dickschichtmembranen kombinieren.

Durch die Einlagerung der stofferkennenden Substanz in die Elektroden werden, im Gegensatz zu Dickschichtmembran-Sensoren, unabhängig vom Volumen der stofferkennenden Substanzen gleichbleibend kurze Ansprechzeiten erzielt. Dies liegt daran, daß die Diffusionsstrecke zwischen dem Ort der chemischen Umsetzung und dem Ort des Nachweises der Umsetzung an der Elektrodenoberfläche überall im Elektrodeninnern minimal ist. Neben kurzen Ansprechzeiten sind bei amperometrischen Sensoren hohe Sensitivitäten erreichbar.

Da das Volumen an stofferkennender Substanz ohne wesentliche Einbußen hinsichtlich der Ansprechzeiten vergrößert werden kann, steht eine hohe Anzahl aktiver Komponenten zur Verfügung. Dadurch wird die Betriebsdauer des Sensors beträchtlich verlängert.

Die einzelnen Teilelektroden stehen flächig in elektrischem Kontakt miteinander, so daß nur eine einzige Teilmatrix mit einer elektrischen Ableitung verbunden zu sein braucht.

Bei Sensoren mit in die Elektrode eingelagerter stofferkennender Substanz kann es vorteilhaft sein, zugunsten der Einlagerung einer größeren Menge der stofferkennenden Substanz bei gleichbleibenden äußeren Elektrodenabmessungen die Hohlraumvolumina zu vergrößern. Auf diese Weise ist eine auf die jeweiligen Anwendungen angepaßte Optimierung von Sensorlebensdauer und -empfindlichkeit bei gleichbleibend kurzen Ansprechzeiten möglich.

Die im wesentlichen dreidimensionale Sensorelektrode steht bevorzugt mit einer auf dem Träger angeordneten elektrischen Ableitung in Kontakt und setzt sich aus mehreren Teilelektroden zusammen (Multilayer-Struktur). In einzelne oder in alle Teilelektroden ist zumindest bereichsweise eine spezifische stofferkennende Substanz, beispielsweise durch Kapillarkräfte, eingebracht. Dabei können in verschiedene Teilelektroden dieselbe oder unterschiedliche stofferkennenden Substanzen eingebettet sein. Die Einbettung ist bei einem Multilayer-Aufbau oftmals einfacher durchführbar als bei massiven Elektroden mit inneren Oberflächen. Auf diese Weise entsteht eine Schichtelektrode, welche eine einfache, schichtweise Herstellung aus einzelnen Teilelektroden gestattet.

Zwischen einzelnen, vorzugsweise mit getrennten Ableitungen versehenen Teilelektroden können aber auch Schichten aus einem elektrisch isolierenden Material angeordnet sein, welches jeweils für den nachzuweisenden Stoff und für reaktionsunterstützende oder reaktionsbegleitende Stoffe permeabel sein kann. Die Teilelektroden können auch durch Abstandsschichten (Spacer) voneinander getrennt sein, welche in einem zentralen Elektrodenbereich Durchbrüche aufweisen. Der Sandwichaufbau gestattet es, in unterschiedliche Teilelektroden unterschiedliche stofferkennende Substanzen einzulagern und simultan eine Mehrzahl von Stoffen in einer Probelösung nachzuweisen.

Die Elektrode oder Teilelektroden können aus einem leitenden Grundkörper, beispielsweise aus Metall, oder aus einem leitfähig beschichteten oder metallisierten, nichtleitenden Grundkörper bestehen. Geeignete leitfähige Grundkörper sind beispielsweise aus Metallen wie Platin, Silber oder Gold oder aus einer Paste, die Kohlenstoff und/oder eines der vorgenannten Metalle enthält. Diese Stoffe eignen sich auch als leitfähige Beschichtung für nichtleitende Grundkörper.

Metallische Grundkörper mit innerer Oberfläche lassen sich beispielsweise durch Ätzen oder durch Laserbehandlung herstellen. Nichtleitende Grundkörper weisen dagegen oft bereits von vorneherein eine innere Oberfläche auf. Als nichtleitende Grundkörper für Elektrode oder Teilelektroden eignen sich Papiere wie Filterpapier, Pappen, Glasfasern, Kunstoffasern, Textilien, Keramiken, mineralische Materialien oder Materialien pflanzlicher oder tierischer Herkunft. Das Aufbringen der Metallisierung kann beispielsweise durch Sputtern, Bedampfen unter Verwendung von Pasten oder Klebern oder durch chemische Reaktion erfolgen.

Vorzugsweise besteht die dreidimensionale Elektrode aus einem isolierenden Grundkörper, welcher möglichst vollständig von einer sehr dünnen Schicht eines Leiters umgeben ist. Durch Minimierung der Metallisierungsdicke lassen sich die Herstellungskosten des Sensors wesentlich reduzieren. Ferner ist es möglich, größere Flächen des Grundkörpers (z.B. Papierbahnen) gleichzeitig und gleichmäßig mit einer Metallisierung zu versehen. Kleinere Stücke davon werden dann als Elektrode oder Teilelektrode für die Sensoren verwendet. Dadurch lassen sich die Herstellungskosten verringern und die Signal-Reproduzierbarkeit steigern.

Die in die Sensorelektrode eingelagerte stofferkennende Substanz enthält bevorzugt mindestens eine aktive Komponente wie Enzyme, Mikroben, Bakterien oder Hefen, welche bevorzugt unter Verwendung von mindestens einem gelartigen oder weichgemachten Polymer wie Polyvinylalkohol, Polyvinylchlorid, Polyurethan, Acrylat oder Silicon immobilisiert wird. Die stofferkennende Substanz kann in den Elektrodengrundkörper durch Ausnutzen der Kapillarwirkung, durch Vakuuminfiltration oder durch Druckbefüllung eingebracht werden.

Auf dem Sensorträger oder im Träger oder zumindest teilweise im Träger des elektrochemischen Sensors sind vorteilhafterweise ein oder mehrere, die mindestens eine Elektrode aufnehmende dreidimensionale Elektrodenräume angeordnet. Dieser mindestens eine Elektrodenraum kann vorteilhafterweise mit mindestens einer Abdeckung versehen sein. Dadurch kann die Elektrode auf einfache Weise und daher kostengünstig schützend verkapselt und auf dem Träger fixiert werden. Diese Verkapselungsmethode vermeidet auch das Problem der Membranhaftung, welches bei Sensoren auf der Basis von Dünnschichtmembranen häufig auftritt.

Der Träger und/oder mindestens eine der Abdeckungen weist vorteilhafterweise eine Öffnung auf, welche ein Eintreten der nachzuweisenden Substanz in den Elektrodenraum gestattet. Durch geeignete Wahld des Öffnungsdurchmessers läßt sich das Sensorverhalten optimieren. Ein geringer Öffnungsdurchmesser beipielsweise verringert das Herausdiffundieren der nachzuweisenden und der umgesetzten Substanz.

Vorteilhafterweise bestehen Träger und Abdeckung aus einem Folienmaterial auf der Basis von beispielsweise Polyethylen, Polyester, Polivinylchlorid, Polypropylen, Polytertrafluorethylen, Celluloseacetat, Silicon oder eine Kombination dieser Stoffe. Das Material sollte für reaktionsunterstützende oder -begleitende Stoffe permeabel, für den zu bestimmenden Stoff jedoch undurchlässig sein. Auf diese Weise wird der lineare Meßbereich des elektrochemischen Sensors erweitert, da eine Verarmung der reaktionsuntersützenden oder -begleitenden Stoffe, d.h. eine Substratlimitierung, hinausgezögert wird. Als Material für Träger und Abdeckung eignen sich besonders gut Folien wie Heißklebefolien, Laminierfolien, Selbstklebefolien oder siegelbare Folien die durch Verschmelzen oder Verkleben miteinander verbunden werden.

Eine auf dem Träger angeordnete und die Elektrode kontaktierende elektrische Ableitung besteht bevorzugt aus einem Metall wie Platin, Silber oder Gold oder aus einer Paste die Kohlenstoff und/oder eines der vorgenannten Metalle enthält, und kann im Siebdruckverfahren, im Schablonendruckverfahren, im Dispensierverfahren, im Sprühverfahren, durch Bedampfen oder durch Besputtern auf den Träger aufgebracht werden.

Außerdem kann auf dem Träger eine Referenz- oder Gegenelektrode angeordnet sein, um einen Bezug zu haben, der nicht von der Probenmatrix oder den Reaktionsprodukten beeinflußt wird, und um bei voltammetrischen Messungen einen Stromfluß zu ermöglichen. Als Material für diese Elektrode eignet sich beispielsweise eine Silber/Silberchloridpaste.

Meßelektrode sowie Referenz- oder Gegenelektrode können beispielsweise über einen auf dem Träger angeordneten Fließkanal mit dem zu analysierenden Medium in Kontakt treten. Ein Sensor mit integriertem Fließkanal eignet sich besonders für eine Automatisierung des Nachweisverfahrens oder für Langzeitmonitoring. Aber auch ein direkter Kontakt mit dem Probenmedium durch Eintauchen oder Betropfen ist denkbar.

Der Aufbau des erfindungsgemäßen elektrochemischen Sensors gestattet es, auf einfache Weise den Einfluß interferierender Stoffe und damit Querempfindlichkeiten zu minimieren. Dies ist auf zwei Arten möglich. Zum einen kann zwischen zu analysierender Probe und der Elektrode eine Interferenzschutzschicht angeordnet sein, welche durch Größen- und/oder Ladungsausschluß ein Eintreten interferierender Stoffe in den Elektrodenraum verhindert. Derartige Schichten können beispielsweise Polymerschichten aus Silicon, Polytetrafluoraethylen, Polyacrylat, Polyurethan, Celluloseacetat, Nafion, Polycarbonat oder Polyvinylchlorid bestehen. Sie können den Elektrodenraum vollkommen oder auch teilweise, etwa im Bereich der Träger- bzw. Abdeckungsöffnung, auskleiden.

Eine weitere Möglichkeit zur Reduzierung des Einflusses interferierender Stoffe besteht darin, zwischen der Nachweiselektrode sowie der zu analysierender Probe eine weitere Elektrode, vorzugsweise im Bereich der Träger- bzw. Abdeckungsöffnung, anzuordnen und mit einer Spannungsquelle zu verbinden. Durch Anlegen einer Spannung an diese Elektrode können interferierende Stoffe oxidiert oder reduziert werden, wodurch sich Querempfindlichkeiten verringern lassen. Vorteilhafterweise befindet sich die weitere Elektrode in unmittelbarer Nähe der Nachweiselektrode mit inneren Oberflächen und ist durch eine isolierende Schicht oder einen dünnen Spalt von dieser elektrisch getrennt.

Die weitere Elektrode kann wie die Nachweiselektrode ausgestaltet sein, wobei jedoch keine stofferkennende Substanz in die Hohlräume eingelagert wird. Auf diese Weise ist gewährleistet, daß interferierende Stoffe auf ihrem Weg durch die weiter Elektrode zur Nachweiselektrode mit großer Wahrscheinlichkeit an der Oberfläche der weiteren Elektrode reduziert oder oxidiert werden.

Weitere positive Eigenschaften und Vorzüge der Erfindugn ergeben sich aus den nach folgend beschriebenen Ausführungsbeispielen und den Figuren. Es zeigen:
- Fig. 1, 2: einen elektrochemischen Sensor (Aufsicht und Schnitt A-A'),
- Fig. 3, 4, 5: einen elektrochemischen Sensor mit integrierter Referenzelektrode (Aufsicht, Schnitte A-A' und B-B'),
- Fig. 6, 7: einen elektrochemischen Sensor mit Einzelkontaktierung der Teilelektroden (Aufsicht und Schnitt A-A'),
- Fig. 8, 9: einen elektrochemischen Sensor mit integrierter Referenzelektrode in Durchflußanordnung (Aufsicht und Schnitt A-A'),
- Fig. 10, 11: einen elektrochemischen Sensor mit Interferenzschutzschicht (Aufsicht und Schnitt A-A'),
- Fig. 12, 13: einen elektrochemischen Sensor mit besonders hoher Cosubstratpermeabilität (Aufsicht und Schnitt A-A') und
- Fig. 14: die Kalibrierkurve eines erfindungsgemäßen elektrochemischen Sensors mit erhöhter Cosubstratpermeabilität

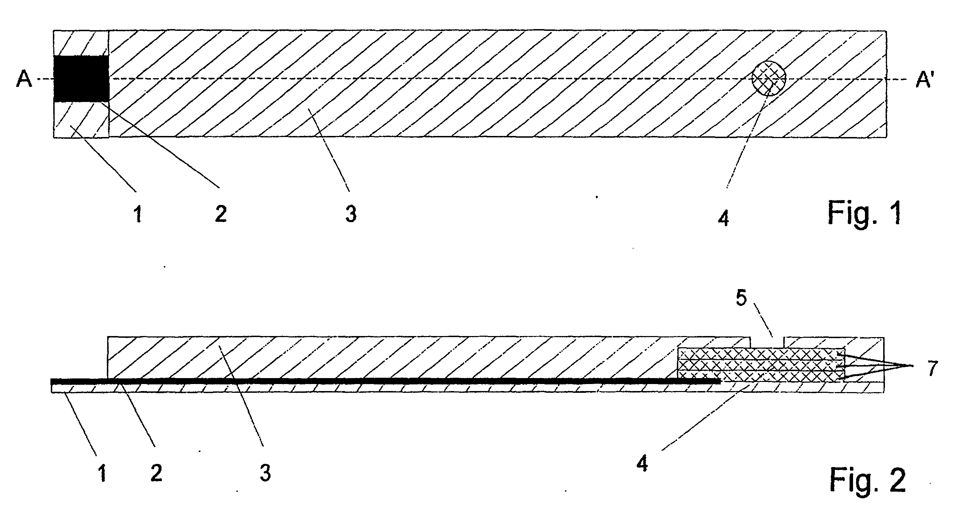

Mit dem in Fig. 1 und Fig. 2 dargestellten elektrochemischen Sensor läßt sich beispielsweise Glucose bestimmen. In diesem Fall wird für den Träger 1 eine 250 µm dicke Laminierfolie aus Polyethylen/Polyester verwendet. Die Ableitung 2 besteht aus einer Kohlenstoffpaste, die mittels Siebdruck auf dem Träger 1 abgeschieden wird und bis in den Elektrodenraum 4 reicht. Im Elektrodenraum 4 befindet sich eine metallisierte Elektrode aus drei mit Platin besputterten dünnen Filterpapieren 7 von 3 mm Durchmesser. Die einzelnen Lagen befinden sich in flächigem elektrischen Kontakt und bilden in ihrer Gesamtheit die dreidimensionale, unregelmäßig netzartige Elektrode. Die Abdeckung 3 besteht aus einer 60 µm dicken Laminierfolie aus Polyethylen/Polyester, die ganzflächig permeabel für Sauerstoff ist, während sie für Glucose nur durch die zuvor gestanzte Öffnung 5 mit einem Durchmesser von 1 mm durchlässig ist. Über diese Öffnung ist die Diffusion von Glucose in den Elektrodenraum 4 möglich. Die stofferkennende Membran, die aus in einer Lösung von Glucoseoxidase in einem photovernetzbaren Polyvinylalkohol-Gel besteht, wird durch die Öffnung 5 in die Poren des metallisierten Elektrodenraums 4 eingebracht. Hierbei erfolgt die Verteilung der Membran im Elektrodenraum 4 durch Kapillarkräfte. Die stofferkennende Membran wird durch UV-Strahlung polymerisiert und somit wasserunlöslich gemacht. Der Elektrodenraum 4 stellt für die stofferkennende Membran ein Reservoir dar und verlängert so die Lebensdauer des Sensors.

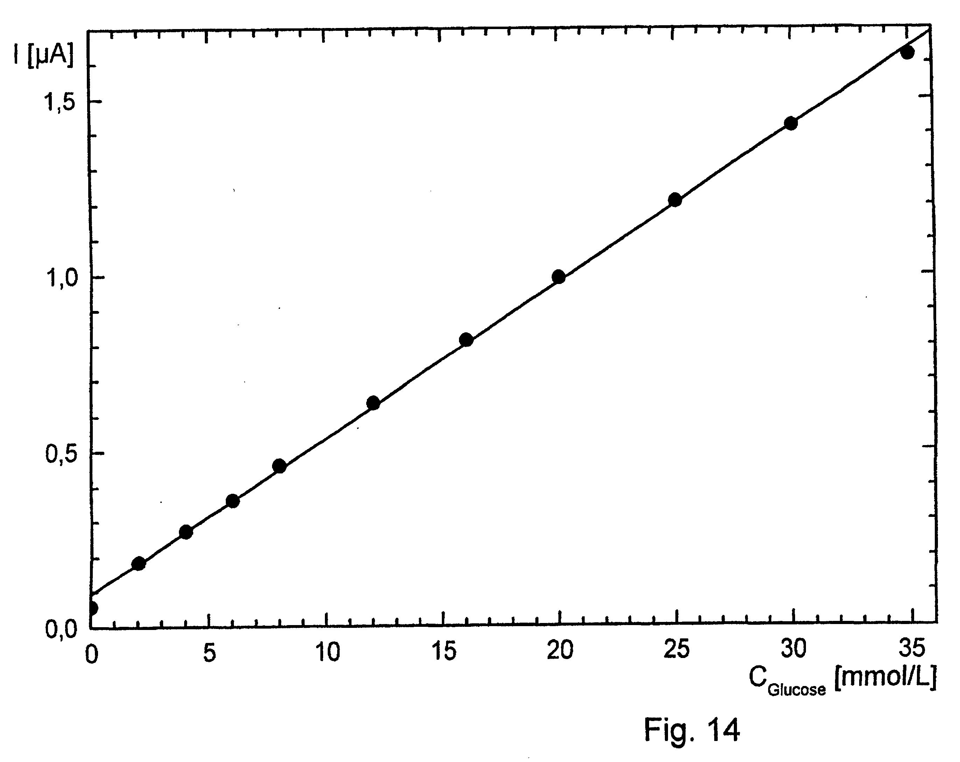

In Fig. 14 ist die Kalibrierkurve eines derartigen Sensors dargestellt. Deutlich zu erkennen ist die gute Linearität auch für hohe Glucosekonzentrationen bis 35 mmol/l aufgrund der verbesserten Cosubstratpermeabilität. Bei herkömmlichen Sensoren liegt das Ende des linearen Bereichs bei wesentlich geringeren Glucosekonzentrationen.

Mit einem derartigen elektrochemischen Sensor ist neben der Glucose-Bestimmung die Bestimmung einer Vielzahl weiterer Substanzen möglich, wobei im wesentlichen die Zusammensetzung der stofferkennenden Membran anzupassen ist. In der folgenden Tabelle sind einige Beispiele skizziert:

| Analyt | stofferkennende Substanz | Cosubstrat | Detektion |
|---|---|---|---|
| Cholesterin | Cholesterinoxidase | Sauerstoff | Wasserstoffperoxid |
| Triglyceride | Esterase, Glycerokinase, Glycerinphosphatoxidase | Sauerstoff | Wasserstoffperoxid |
| Creatinin | Creatininase, Creatinase, Sacrosinoxidase | Sauerstoff | Wasserstoffperoxid |
| Harnsäure | Uricase | Sauerstoff | Wasserstoffperoxid |
| | | | |

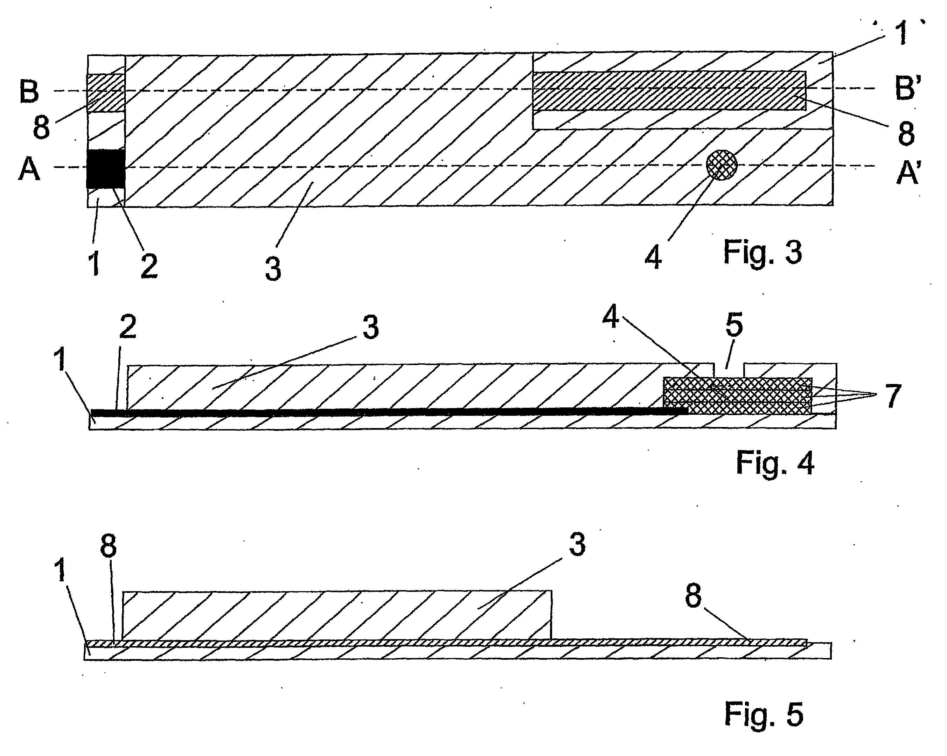

Die Integration der Gegenelektrode 8 ist in Figur 3, 4 und 5 dargestellt. Bei einer Zweielektrodenanordnung übernimmt die Gegenelektrode 8 auch die Funktioin der Referenzelektrode. Als Material hierfür dient eine Silber/Silberchloridpaste, die mittels Siebdruck auf den Träger 1 aufgetragen wird.

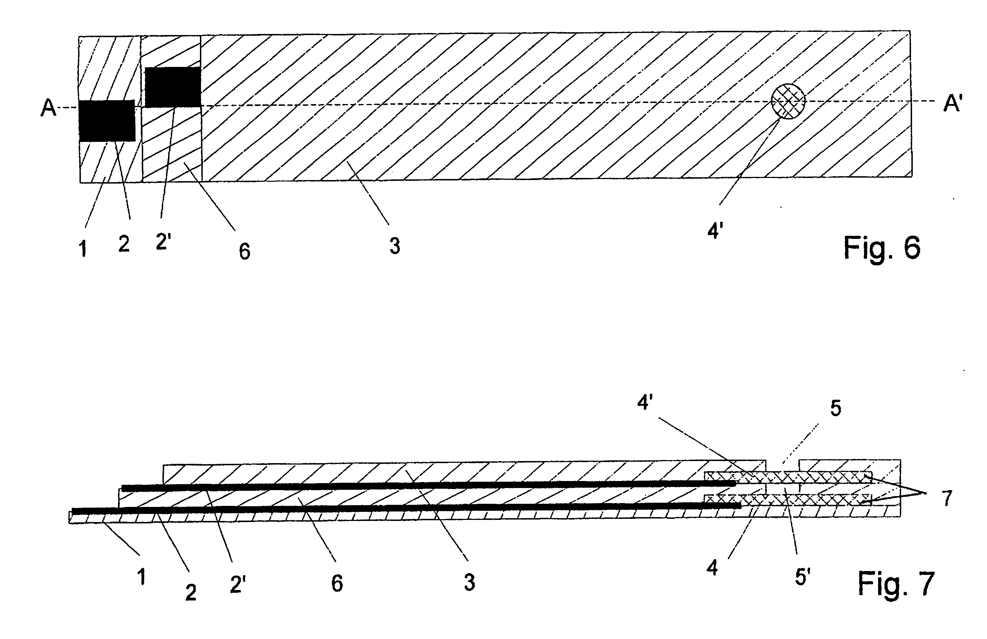

Als nächstes Beispiel der Erfindung ist in Figur 6 und 7 die Kontaktierung von einzelnen Elektrodenräumen 4 und 4' durch getrennte Ableitungen 2 und 2' dargestellt. Die Ableitungen 2 und 2' sind durch eine Isolationsschicht (Spacer) 6 voneinander getrennt.

Durch die Öffnung 5' in der Isolationsschicht 6 sind die getrennten Elektrodenräume 4 und 4' miteinander verbunden. Mit dieser Sensorkonfiguration kann man zwei Stoffe gleichzeitig nachweisen, indem in die Elektrodenräume 4 und 4' unterschiedliche stofferkennende Membranen eingebracht werden.

Außerdem besteht die Möglichkeit, lediglich die Elektrode in Elektrodenraum 4 mit einer stofferkennenden Membran auszufüllen. Für das Beispiel der Glucose-Bestimmung dient die im Elektrodenraum 4' eingelagerte und mit einer Spannungsquelle verbundene Elektrode dann dazu, oxidierbare Substanzen wie Ascorbinsäure oder Harnsäure bei 700 mV gegen eine Silber/Silberchloridpaste zu oxidieren, so daß sie bei der Bestimmung im Elektrodenraum 4 nicht mehr stören.

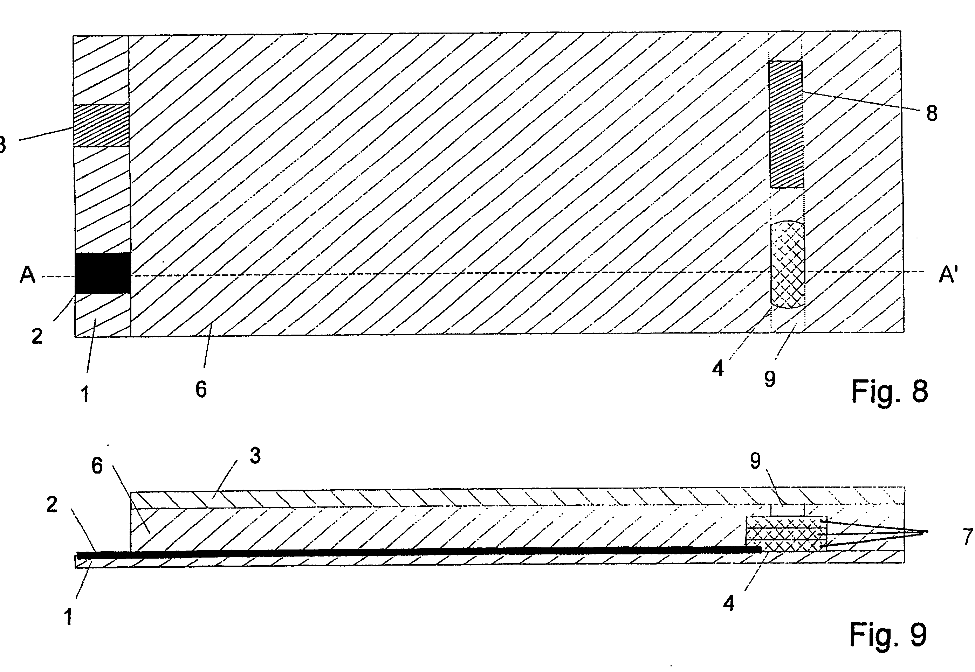

Eine weitere Modifikation der Elektrode ist in Figur 8 und 9 dargestellt. Hier wird zwischen dem Elektrodenraum 4 sowie der Referenzelektrode 8 und der Abdeckung 3 ein Fließkanal 9 eingefügt, wodurch eine Adaptierung des Sensors für Durchflußmessungen ohne großen Aufwand ermöglicht wird.

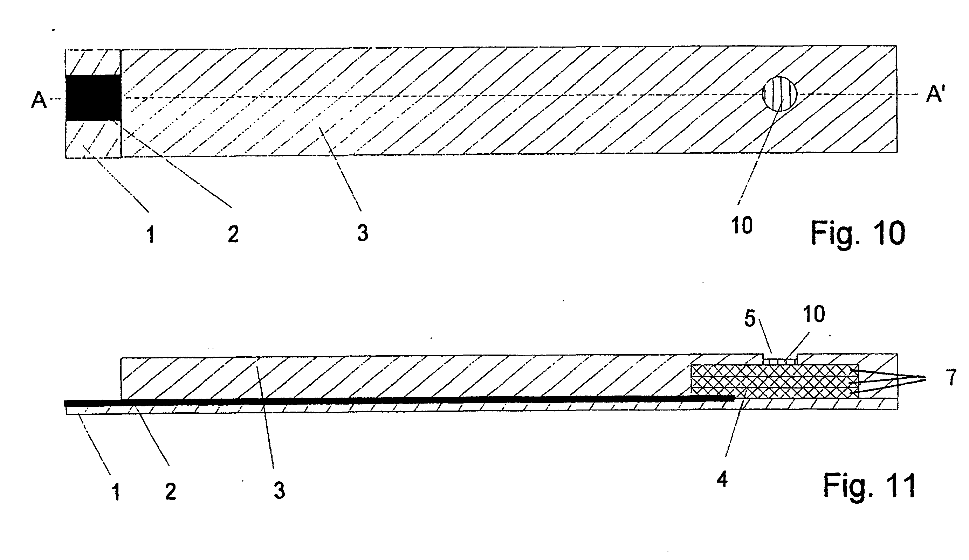

In Figur 10 und 11 ist einer weitere erfindungsgemäße Elektrode dargestellt, bei der oberhalb des Elektrodenraumes 4 eine Interferenzschutzschicht aufgebracht ist.

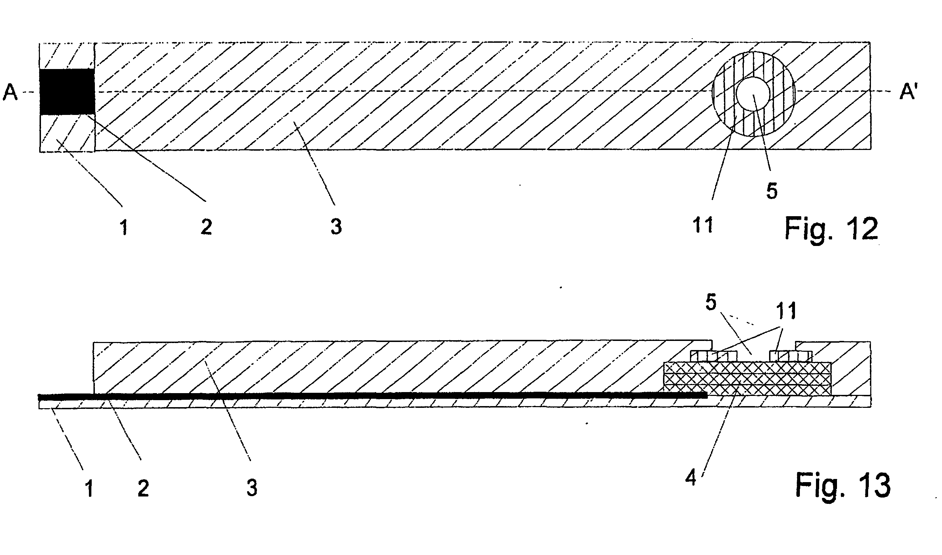

In Figur 12 und 13 ist ein weiteres Beispiel des erfindungsgemäßen Sensors dargestellt. In die Abdeckung 3 aus Laminierfolie wird ein größeres Loch als Öffnung 5 des Elektrodenraumes 4 gestanzt. Der Elektrodenraum 4 wird von einer Schicht 11, welche eine besonders hohe Cosubstratpermeabilität aufweist und inder die Öffnung 5 eingebracht ist, abgedeckt. Für den Glucosesensor wird hierfür 30 µm dünne Silikonfolie verwendet, ein Material mit sehr hoher Sauerstoffpermeabilität. Auf diese Weise kann man auch Materialien, die sich nicht besonders leicht verschmelzen oder verkleben lassen, für den Sensoraufbau verwenden.

## Patentansprüche

1. Elektrochemischer Sensor zur Bestimmung von Stoffkonzentrationen mit einem Träger (1) und mindestens einer, im Bereich des Trägers (1) angeordneten Elektrode, die im Elektrodeninnern Hohlräume aufweist, in die zumindest bereichsweise eine stofferkennende Substanz eingelagert ist,
**dadurch gekennzeichnet,**
**daß** die mindestens eine Elektrode aus mindestens zwei Teilelektroden (7) in Form von übereinander angeordneten Schichten besteht.

2. Elektrochemischer Sensor nach Anspruch 1, **dadurch gekennzeichnet, daß** zwischen Teilelektroden (7) Schichten (6) aus einem elektrisch isolierenden Material angeordnet sind.

3. Elektrochemischer Sensor nach Anspruch 1 oder 2,
**dadurch gekennzeichnet, daß** isolierte Teilelektroden (7) mit separaten Ableitungen (2, 2') versehen sind.

4. Elektrochemischer Sensor nach mindesten einem der vorhergehenden Ansprüche, **dadurch gekennzeichnet, daß** die Teilelektroden (7) aus einem elektrisch leitenden Grundkörper besteht.

5. Elektrochemischer Sensor nach Anspruch 4, **dadurch gekennzeichnet, daß** die Teilelektroden (7) aus einem leitfähig beschichteten, nichtleitenden Grundkörper besteht.

6. Elektrochemischer Sensor nach Anspruch 4 oder 5,
**dadurch gekennzeichnet, daß** der leitende Grundkörper oder die leitfähige Beschichtung Platin, Silber, Gold oder Kohlenstoff oder eine Paste aus einem oder mehreren dieser Stoffe enthält.

7. Elektrochemischer Sensor nach einem der Ansprüche 5 oder 6, **dadurch gekennzeichnet, daß** der Grundkörper der Teilelektroden (7) aus Papier, Filterpapier, Pappe, Glasfasern, Kunststoffasern, Textil, Keramik, mineralischem Material oder aus Material pflanzlicher oder tierischer Herkunft besteht.

8. Elektrochemischer Sensor nach mindestens einem der vorhergehenden Ansprüche, **dadurch gekennzeichnet, daß** die stofferkennende Substanz mindestens eine der Komponenten Enzyme, Mikroben, Bakterien oder Hefen enthält.

9. Elektrochemischer Sensor nach mindestens einem der vorhergehenden Ansprüche, **dadurch gekennzeichnet, daß** die stofferkennende Substanz mit Hilfe mindestens eines gelartigen oder weichgemachten Polymers wie Polyvinylalkohol, Polyvinylchlorid, Polyurethan, Acrylat oder Silikon immobilisiert ist.

10. Elektrochemischer Sensor nach mindestens einem der vorhergehenden Ansprüche, **dadurch gekennzeichnet, daß** auf dem, im oder teilweise im Träger (1) ein oder mehrere, die mindestens eine Elektrode oder die Teilelektroden (7) aufnehmende Elektrodenräume (4, 4') angeordnet sind.

11. Elektrochemischer Sensor nach mindestens einem der vorhergehenden Ansprüche, **dadurch gekennzeichnet, daß** der mindestens eine Elektrodenraum (4, 4') mit mindestens einer Abdeckung (3, 11) versehen ist.

12. Elektrochemischer Sensor nach mindestens einem der vorhergehenden Ansprüche, **dadurch gekennzeichnet, daß** der Träger (1) und/oder die mindestens eine Abdeckung (3, 11) für reaktionsunterstützende oder begleitende Stoffe permeabel und für den jeweils zu bestimmenden Stoff undurchlässig sind.

13. Elektrochemischer Sensor nach mindestens einem der vorhergehenden Ansprüche, **dadurch gekennzeichnet, daß** der Träger (1) und/oder die mindestens eine Abdeckung (3, 11) mindestens eine Öffnung (5, 5') aufweist.

14. Elektrochemischer Sensor nach mindestens einem der vorhergehenden Ansprüche, **dadurch gekennzeichnet, daß** der Träger (1) und/oder die mindestens eine Abdeckung (3, 11) aus einer Heißklebefolie, einer Laminierfolie, einer Selbstklebefolie oder einer siegelbaren Folie bestehen.

15. Elektrochemischer Sensor nach mindestens einem der vorhergehenden Ansprüche, **dadurch gekennzeichnet, daß** auf dem Träger (1) eine Referenzund/oder Gegenelektrode (8) angeordnet ist.

16. Elektrochemischer Sensor nach mindestens einem der vorhergehenden Ansprüche, **dadurch gekennzeichnet, daß** die Elektrode und bei der Ausgestaltung nach Anspruch 15 zusätzlich die Referenz- und/oder Gegenelektrode (8) über einen Fließkanal (9) mit dem zu analysierenden Probemedium in Kontakt treten.

17. Elektrochemischer Sensor nach mindestens einem der vorhergehenden Ansprüche, **dadurch gekennzeichnet, daß** zwischen dem zu analysierendem Probenmedium und einer Teilelektrode eine Interferenzschutzschicht (10) angeordnet ist.

18. Elektrochemischer Sensor nach mindestens einem der vorhergehenden Ansprüche, **dadurch gekennzeichnet, daß** die Interferenzschutzschicht (10) eine Polymerschicht aus Silikon, Polytetrafluorethylen, Polyacrylat, Polyurethan, Celluloseacetat, Nafion, Polycarbonat oder Polyvinylchlorid ist.

19. Elektrochemischer Sensor nach mindestens einem der vorhergehenden Ansprüche, **dadurch gekennzeichnet, daß** zwischen zu analysierendem Probemedium und einer Teilelektrode eine weitere Elektrode oder Teilelektrode angeordnet ist, welche mit einer Spannungsquelle verbunden ist.

20. Elektrochemischer Sensor nach Anspruch 19, **dadurch gekennzeichnet, daß** die weitere Elektrode im Bereich der Öffnung (5, 5') der Abdeckung (3, 11) angeordnet ist.

21. Elektrochemischer Sensor nach mindestens einem der vorhergehenden Ansprüche, **dadurch gekennzeichnet, daß** die stofferkennende Membran Glucoseoxidase zur Bestimmung von Glucose enthält und der Träger oder die Abdeckung durchlässig für Sauerstoff ist.

## Claims

1. An electrochemical sensor for determining concentrations of substances, comprising a support (1) and at least one electrode which is arranged in the region of the support (1) and has cavities inside the electrode, the cavities being at least partly filled with a substance-detecting agent, **characterised in that** the at least one electrode comprises at least two sub-electrodes (7) in the form of superimposed layers.

2. An electrochemical sensor according to claim 1, **characterised in that** layers (6) of an electrically insulating material are arranged between sub-electrodes (7).

3. An electrochemical sensor according to claim 1 or 2,
**characterised in that** insulated sub-electrodes (7) are provided with separate conductors (2, 2').

4. An electrochemical sensor according to at least one of the preceding claims, **characterised in that** the sub-electrodes (7) comprise an electrically conducting main body.

5. An electrochemical sensor according to claim 4, **characterised in that** the sub-electrodes (7) comprise a conductively coated, nonconducting main body.

6. An electrochemical sensor according to claim 4 or 5,
**characterised in that** the conducting main body or the conductive coating contains platinum, silver, gold or carbon or a paste comprising one or more of these materials.

7. An electrochemical sensor according to either one of claims 5 and 6, **characterised in that** the main body of the sub-electrodes (7) comprises paper, filter paper, board, glass fibres, plastics fibres, textile, ceramic, mineral material or material of vegetable or animal origin.

8. An electrochemical sensor according to at least one of the preceding claims, **characterised in that** the substance-detecting agent contains at least one of the components enzymes, microbes, bacteria or yeasts.

9. An electrochemical sensor according to at least one of the preceding claims, **characterised in that** the substance-detecting agent is immobilised with the aid of at least one gel-like or plasticised polymer such as polyvinyl alcohol, polyvinyl chloride, polyurethane, acrylate or silicone.

10. An electrochemical sensor according to at least one of the preceding claims, **characterised in that** one or more electrode spaces (4, 4'), holding the at least one electrode or the sub-electrodes (7), are provided on, in or partly in the support (1).

11. An electrochemical sensor according to at least one of the preceding claims, **characterised in that** the at least one electrode space (4, 4') is provided with at least one covering (3, 11).

12. An electrochemical sensor according to at least one of the
preceding claims, **characterised in that** the support (1) and/or the at least one covering (3, 11) are permeable to reaction-assisting or accompanying substances and impermeable to the respective substance to be identified.

13. An electrochemical sensor according to at least one of the preceding claims, **characterised in that** the support (1) and/or the at least one covering (3, 11) have at least one opening (5, 5').

14. An electrochemical sensor according to at least one of the preceding claims, **characterised in that** the support (1) and/or the at least one covering (3, 11) comprise a heat-sealing film, a laminating film, a self-adhesive film or a sealable film.

15. An electrochemical sensor according to at least one of the preceding claims, **characterised in that** a reference and/or counterelectrode (8) is arranged on the support (1).

16. An electrochemical sensor according to at least one of the preceding claims, **characterised in that** the electrode and, in the embodiment according to claim 15, also the reference and/or counterelectrode (8) come into contact with the test medium to be analysed via a flow channel (9).

17. An electrochemical sensor according to at least one of the preceding claims, **characterised in that** an interference protective layer (10) is arranged between the test medium to be analysed and a sub-electrode.

18. An electrochemical sensor according to at least one of the preceding claims, **characterised in that** the interference protective layer (10) is a polymer layer of silicone, polytetrafluoroethylene, polyacrylate, polyurethane, cellulose acetate, nafion, polycarbonate or polyvinyl chloride.

19. An electrochemical sensor according to at least one of the preceding claims, **characterised in that** a further electrode or sub-electrode, connected to a voltage source, is arranged between a test medium to be analysed and a sub-electrode.

20. An electrochemical sensor according to claim 19, **characterised in that** the further electrode is arranged in the region of the opening (5, 5') of the covering (3, 11).

21. An electrochemical sensor according to at least one of the preceding claims, **characterised in that** the substance-detecting membrane contains glucose oxidase for detecting glucose, and the support or the covering is permeable to oxygen.

## Revendications

1. Détecteur électrochimique pour déterminer des concentrations de matière comportant un support (1) et au moins une électrode qui est disposée dans la zone du support (1) et qui comporte, à l'intérieur de l'électrode, des cavités dans lesquelles une substance identifiant la matière est insérée au moins par endroits, **caractérisé en ce que** la au moins une électrode est constituée d'au moins deux électrodes (7) partielles sous la forme de couches disposées les unes au-dessus des autres.

2. Détecteur électrochimique selon la revendication 1, **caractérisé en ce que** des couches (6) formées d'un matériau électriquement isolant sont disposées entre des électrodes (7) partielles.

3. Détecteur électrochimique selon la revendication 1 ou 2, **caractérisé en ce que** des électrodes partielles isolées (7) sont équipées de conducteurs de dérivation séparés (2, 2').

4. Détecteur électrochimique selon au moins l'une des revendications précédentes, **caractérisé en ce que** les électrodes (7) partielles sont constituées par un corps de base électriquement conducteur.

5. Détecteur électrochimique selon la revendication 4, **caractérisé en ce que** les électrodes (7) partielles sont constituées par un corps de base non conducteur, pourvu d'un revêtement conducteur.

6. Détecteur électrochimique selon la revendication 4 ou 5, **caractérisé en ce que** le corps de base conducteur ou le revêtement conducteur contient du platine, de l'argent, de l'or ou du carbone ou une pâte formée d'un ou de plusieurs de ces matériaux.

7. Détecteur électrochimique selon la revendication 5 ou 6, **caractérisé en ce que** le corps de base des électrodes (7) partielles est formé de papier, de papier filtre, de carton, de fibres de verre, de fibres de matière plastique, d'un textile, d'une céramique, d'un matériau minéral ou d'un matériau d'origine végétale ou animale.

8. Détecteur électrochimique selon au moins l'une des revendications précédentes, **caractérisé en ce que** la substance identifiant la matière contient au moins l'un des composants: enzymes, microbes, bactéries ou levures.

9. Détecteur électrochimique selon au moins l'une des revendications précédentes, **caractérisé en ce que** la substance identifiant la matière est immobilisée à l'aide d'au moins un polymère en forme de gel ou d'un polymère assoupli, tel qu'un alcool polyvinylique, du chlorure de polyvinyle, du polyuréthane, un acrylate ou du silicone.

10. Détecteur électrochimique selon au moins l'une des revendications précédentes, **caractérisé en ce qu'**une ou plusieurs chambres (4, 4') à électrodes, qui logent au moins une électrode ou les électrodes (7) partielles, sont disposées sur, dans ou partiellement dans le support (1).

11. Détecteur électrochimique selon au moins l'une des revendications précédentes, **caractérisé en ce qu'**au moins une chambre à électrodes (4, 4') est équipée d'au moins un élément de recouvrement (3, 11).

12. Détecteur électrochimique selon au moins l'une des revendications précédentes, **caractérisé en ce que** le support (1) et/ou le au moins un élément de recouvrement (3, 11) sont perméables pour des matières favorisant la réaction ou l'accompagnant, et sont imperméables pour la matière devant être déterminée.

13. Détecteur électrochimique selon au moins l'une des revendications précédentes, **caractérisé en ce que** le support (1) et/ou le au moins un élément de recouvrement (3, 11) possède au moins une ouverture (5, 5').

14. Détecteur électrochimique selon au moins l'une des revendications précédentes, **caractérisé en ce que** le support (1) et/ou le au moins un élément de recouvrement (3, 11) est formé par une feuille adhésive à chaud, une feuille de stratification, une feuille autoadhésive ou une feuille pouvant être scellée.

15. Détecteur électrochimique selon au moins l'une des revendications précédentes, **caractérisé en ce qu'**une électrode de référence et/ou une contre-électrode (8) sont disposés sur le support (1).

16. Détecteur électrochimique selon au moins l'une des revendications précédentes, **caractérisé en ce que** l'électrode et, dans le cas de l'agencement de la revendication 15, en outre l'électrode de référence et/ou la contre-électrode (8) viennent en contact avec le milieu échantillon à analyser, par l'intermédiaire d'un canal d'écoulement (9).

17. Détecteur électrochimique selon au moins l'une des revendications précédentes, **caractérisé en ce qu'**une couche (10) de protection contre les interférences est disposée entre le milieu échantillon à analyser et une électrode partielle.

18. Détecteur électrochimique selon au moins l'une des revendications précédentes, **caractérisé en ce que** la couche (10) de protection contre les interférences est une couche polymère formée de silicone, de polytétrafluoréthylène, de polyacrylate, de polyuréthane, d'acétate de cellulose, de nafion, de polycarbonate ou de chlorure de polyvinyle.

19. Détecteur électrochimique selon au moins l'une des revendications précédentes, **caractérisé en ce qu'**entre le milieu échantillon à analyser et une électrode partielle est disposée une autre électrode ou électrode partielle, qui est reliée à une source de tension.

20. Détecteur électrochimique selon la revendication 19, **caractérisé en ce que** l'autre électrode est disposée dans la zone de l'ouverture (5, 5') de l'élément de recouvrement (3,11).

21. Détecteur électrochimique selon au moins l'une des revendications précédentes, **caractérisé en ce que** la membrane, qui identifie la matière, contient de l'oxydase de glucose servant à déterminer le glucose et le support ou l'élément de recouvrement est perméable pour l'oxygène.
